# EUROPEAN PATENT SPECIFICATION

(11) **EP 4 125 613 B1**
(45) Date of publication and mention of the grant of the patent: **03.07.2024**
(21) Application number: 21714219.9
(22) Date of filing: 29.03.2021
(51) Int. Cl.: A61B 10/00, G01N 33/50, G01N 1/02, A61B 10/02

(54) **FIBROUS MAT FOR METABOLOME SAMPLING**
FASERMATTE ZUR METABOLOM-ENTNAHME
TAPIS FIBREUX POUR UN ÉCHANTILLONNAGE DE MÉTABOLOME

(30) Priority: 27.03.2020 EP 20166132
(43) Date of publication of application: 08.02.2023
(73) Proprietor: Universiteit Gent, 9000 Gent (BE)
(72) Inventor: VANHAECKE, Lynn, 9860 Balegem (BE); DE SPIEGELEER, Margot, 9000 Gent (BE); DE CLERCK, Karen, 9051 Afsnee (BE); GELTMEYER, Jozefien, 9030 Mariakerke (BE); SINGH, Varoon, Palghar, Mumbay 401203 (IN)
(74) Representative: Winger
(86) International application number: PCT/EP2021/058162
(87) International publication number: WO 2021/191467

(56) References cited:
- WO-A1-2016/142691
- WO-A1-2017/209628
- WO-A2-2007/054040
- US-A1- 2012 045 752

## Description

### Field of the invention

The invention relates to the field of metabolome sampling and desorption. In particular to the field of metabolome sampling, desorption and ionization. More specifically, it relates to a device and method for taking a metabolome sample and for desorption of the sampled metabolites or for desorpion and ionization of the sampled metabolites.

### Background of the invention

To date, advancements in sophisticated chemical analytical platforms and informatic tools have made it possible to simultaneously measure thousands of metabolites in body fluids such as blood and urine. Profiling and quantification of biomolecules are best achieved by high-resolution mass spectrometry (HRMS) conveniently hyphenated with chromatographic separation techniques such as liquid chromatography (LC). In specific, metabolic profiling of fecal samples has been carried out using gas chromatography (GC)/MS, LC/MS and nuclear magnetic resonance (NMR) spectroscopy. However, these powerful metabolomics platforms for biomarker discovery are very expensive and laborious. Moreover, they impose large instrumentation of which the analytical results can only be handled by (bio)analytical experts in the field of MS-based metabolomics, and the required extensive sample pretreatment (extraction and preparation protocols) before analysis is associated with the risk of metabolite alterations or loss during processing steps and, in addition, can induce potential unwanted selectivity to the analysis resulting thereof. Therefore, the translation of current hyphenated HRMS techniques into large-scale screening of very large populations in providing sufficient statistical power for confident biomarker assessment and in point-of-care (POC) applications as well, which requires in situ testing, is very unlikely.

US2012/045752 A1 relates to a bioparticle collection device and an aerosol collection system. The device includes a collection medium including a plurality of fibers formed in a fiber mat and configured to collect bioparticles thereon. This fiber mat is positioned in a gas stream induced by an aerosol pumping device.

WO 2017/209628 relates to an improved evidence collection and storage apparatus which comprises a container and a swab that can be used for collection, storage and analysis of a sample Also disclosed is a mat as defined in the preamble of claim 1.

WO 2007/054040 relates to filters for removing physical and/or biological impurities.

Sample pretreatment involves the separation of analytes and/or components including compounds of interest from complex biological matrices and is traditionally performed by liquid-liquid extraction protocols regarding the analysis of the metabolome. Such methods are time consuming, difficult to automate and require significant sample consumption and high purity, expensive organic solvents generating substantial chemical waste. Similarly, solid phase extraction is a time-demanding preconcentration step. Therefore, ambient ionization approaches (hyphenated to MS) that desorb/ionize analytes and/or components including compounds directly, generating gas-phase compounds/ions from the crude sample surface in the open air and enabling subsequent introduction into the mass spectrometer allow to circumvent sample preparation and chromatographic separation, thereby enabling in situ, rapid, low-cost, operationally simple POC analysis. The operational simplicity, speed (e.g. < 10 seconds per sample) and limited cleaning requirements in between samples further support the translation of MS-information into direct (on-site) personalized healthcare decisions for future POC application.

Presently in metabolomics studies on stool samples, it is common practice to collect whole fecal samples, which is highly dependent on individual fecal urge and often is contaminated with urine and/or toilet water comprising further analysis. Also, collected samples will need to be stored immediately and accurately in the patients' freezer and urge transportation to an analytical laboratory. Subsequent analysis is then subject to labor-intensive and time-consuming extraction protocols, imparting both experimental as technical variations, prior to analysis by LC hyphenated MS towards generating qualitative and quantitative (biased) results. Additionally, liquid-liquid extractions and other analytical methods have been developed and optimized for a specific fraction of the metabolome, thereby introducing unwanted selectivity.

When, by way of example, considering clinical applications in children, measurements such as the homeostatic model assessment of insulin resistance (HOMA-IR) and Matsuda index are most frequently performed to investigate IR in children. These approaches impart the withdrawal of blood, which imposes both pain and stress on the child. However, stress (e.g. cortisol) is deemed a contributing factor in the development of metabolic diseases, and as such the upstream analysis of the blood withdrawn might be biased already at this stage. Next to this, the current procedure to diagnose cow's milk allergy entails the clinical history of the patient, followed by several challenge-based diagnostic tests, of which the most accurate is the double-blind, placebo-controlled food challenge. However, this test is expensive, time consuming and may lead to severe allergic reactions. An alternative to this test is the measurement of immunoglobulin E (IgE)-antibodies in a venous blood sample or through a skin prick test. The sensitivity of these tests is very high, but 1 in 4 children receive false positive results, leading to unnecessary imposement of restrictive diets.

Regarding the analysis of biological samples in minimizing sample preparation, matrix assisted laser desorption ionization (MALDI)-MS and shotgun metabolomics are common techniques, in which the prior separation by G/LC is eliminated. However, the former infers the usage of an organic matrix which is a substantial drawback in the detection of small molecules (causing significant background interference in the low mass range < 1000 Da) and furthermore, introduces the risk of artifact formation and has crystal-size-related resolution limits, which in turn causes poor reproducibility. Regarding the latter technique, i.e. shotgun metabolomics, troublesome matrix effects have been reported nor was it able to distinguish isomeric analytes and/or components including compounds.

There is therefore a need for a good device and for a good method for taking and releasing metabolome samples.

### Summary of the invention

It is an object of embodiments of the present invention to provide a good material/device and to provide a good method for taking and releasing metabolome samples.

The above objective is accomplished by a method and device according to the present invention.

In a first aspect embodiments of the present invention relate to a fibrous mat as defined in claim 1.

It is an advantage of embodiments of the present invention that only metabolites (with a size smaller than 2000 Da) are extracted from the biofluid and adsorbed and/or absorbed to/in the open network of one or more nanofiber(s). It is found by the inventors that particles with a size larger than 2000 Dalton can be washed away from the cover layer and that the metabolites will remain adsorbed and/or absorbed to/in the open network when washing away particles present in/on the cover layer.

The desorption may for example be laser-assisted desorption. The desorption may for example be thermally activated (e.g. radiative and Joule heating mechanism).

The desorption may be followed by or combined with ionization of the metabolome sample. In embodiments of the present invention the fibrous mat is for use in ionization of the metabolome sample such as laser-assisted ionization of the metabolome sample. Any ionization technique known in the art may be used for ionization of the metabolome sample. For example, electrospray ionization (ESI), or desorption electrospray ionization (DESI).

In embodiments of the present invention the open network comprises interconnected pores.

In embodiments of the present invention the fibrous mat comprises nano- or micro-particles which are incorporated in the open network. The nano- or micro-particles may be incorporated in the at least one nanofiber or embedded in the open network formed by the nanofiber.

In embodiments of the present invention the fibrous mat is partly hydrophobic and partly hydrophilic.

In embodiments of the present invention the at least one nanofiber is comprising one or more materials selected from:
a list of hydrophilic polymers comprising polyacrylate, polyacrylonitrile, polyvinylpyrrolidone (PVP),
and/or from a list of hydrophobic polymers comprising polystyrene (PS), divinylbenzene, polydimethylsiloxane, polydivinylbenzene,
and/or from a list of combined hydrophobic/hydrophilic polymers comprising polydivinylbenzene-co-polyvinylpyrrolidone, hydrophilic divinylbenzene, crosslinked polyvinylpyrrolidone, polyaniline.

In embodiments of the present invention the open network of nanofibers may comprise PVP/PS nanofibers. These may be covered with a PAN cover layer.

Embodiments of the present invention may also relate to a sampling device comprising a fibrous mat according to embodiments of the present invention and a carrier to which the fibrous mat is secured.

In a second aspect embodiments of the present invention relate to a method for desorption of a metabolome sample, as defined in claim 10.

In embodiments of the present invention the desorption may be combined with the ionisation of the metabolome sample. This may be achieved through laserassisted ionization.

In a method according to embodiments of the present invention the provided fibrous mat is a mat according to embodiments of the present invention.

Embodiments of the present invention relate to a method for providing a fibrous mat, as defined in claim 14.

In embodiments of the present invention the at least one polymer is spun such that interconnected pores are formed between the nanofibers, for example between entangled nanofibers. Where in embodiments of the present invention reference is made to entangled nanofibers, reference is made to a non-woven distribution of nanofibers. Preferably no physical connection is present between the entangled nanofibers. There may, however, be a physical connection between the entangled nanofibers.

In embodiments of the present invention the electrospinning is done on a carrier, such as polyurethane for example.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### Brief description of the drawings

FIG. 1 shows a schematic drawing of a sampling device and of an open network of at least one polymer-based fibrous mat in accordance with embodiments of the present invention.
FIG. 2 shows a flow chart of a method for desorption of a metabolome sample in accordance with embodiments of the present invention.
FIG. 3 shows a SEM image of an open network of at least one polymer-based nanofiber of a fibrous mat in accordance with embodiments of the present invention.
FIG. 4 shows a SEM image of a nanofiber used in a fibrous mat in accordance with embodiments of the present invention.
FIG. 5 shows a SEM image of an open network of at least one polymer-based nanofiber of a fibrous mat wherein microparticles are embedded in the open network, in accordance with embodiments of the present invention.
FIG. 6 shows a SEM image of a PVP/PS hydrophilic/hydrophobic fibrous mat with phase separation, in accordance with embodiments of the present invention.
FIG. 7 illustrates the method steps and the use of a fibrous mat for taking a metabolome sample and for desorption or desorption and ionization and high-resolution mass spectrometric (HRMS) analysis of the metabolome sample.
FIG. 8 is a graph illustrating the metabolome coverage of different fibrous mats in accordance with embodiments of the present invention compared to feces as such.
FIG. 9 shows a validated multivariate (OPLS-DA) model from stool of CMA children on a fibrous mat, in accordance with embodiments of the present invention.
FIG. 10 shows a SEM image of a fibrous mat comprising a PAN cover layer over a PVP/PS layer, in accordance with embodiments of the present invention.
FIG. 11 shows a SEM image of an open network of PVP/PS fibers of a fibrous mat, in accordance with embodiments of the present invention.
FIG. 12 shows a SEM image of PAN fibers in a cover layer of a fibrous mat, in accordance with embodiments of the present invention.
FIG. 13 shows a chemical drawing illustrating chemical interactions of nanofiber moieties, of a fibrous mat in accordance with embodiments of the present invention, with example analytes of different polarity (log P range = -2.5 to 14.5).
FIG. 14 and FIG. 15 show the mass spectra of faeces obtained using a fibrous mat and without using a fibrous mat, in accordance with embodiments of the present invention.
FIG. 16 and FIG. 17 show the mass spectra of saliva obtained using a fibrous mat and without using a fibrous mat, in accordance with embodiments of the present invention.
FIG. 18 and 19 shows the mass spectra of urine obtained using a fibrous mat and without using a fibrous mat, in accordance with embodiments of the present invention.
FIG. 20 shows the mass spectral feature count for different fibrous mat compositions with PAN under- and/or overcoated, in accordance with embodiments of the present invention.
FIG. 21 shows a SEM image of an open network of at least one polymer-based nanofiber of a fibrous mat, in accordance with embodiments of the present invention, additionally comprising microparticles incorporated as a part of one or more nanofibers.

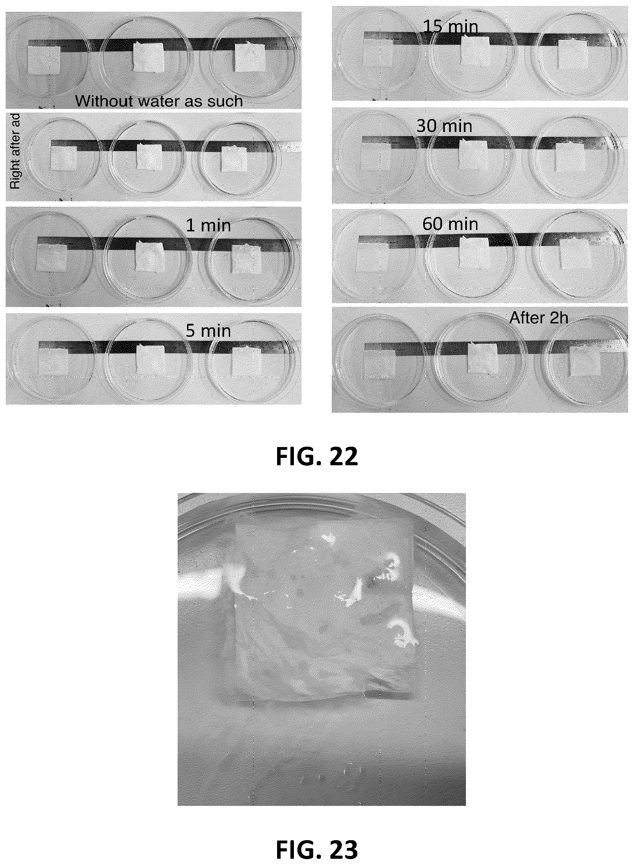

The pictures in FIG. 22 show measurement setups for measuring the deformation of the fibrous mat.

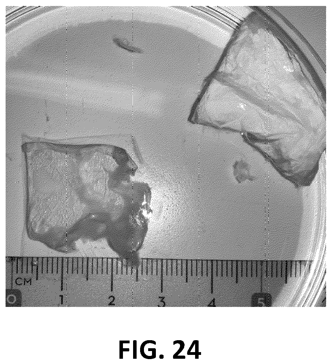

FIG. 23 and FIG. 24 show SEM images of non-coherent fibrous mats which are falling apart and dissolved due to the fact that they are submerged in water.

Any reference signs in the claims shall not be construed as limiting the scope. In the different drawings, the same reference signs refer to the same or analogous elements.

### Detailed description of illustrative embodiments

The present invention will be described with respect to particular embodiments and with reference to certain drawings, but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some, but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

Where in embodiments of the present invention reference is made to an open network, reference is made to a network in which the pores are interconnected and/or the nanofibers are entangled. With interconnected pores is meant that the pores from a passage. This passage may be from one side of the open network to the other side of the open network. This is, however, not strictly required.

In a first aspect embodiments of the present invention relate to a fibrous mat 100 for use in taking a metabolome sample and for use in desorption of the metabolome sample. The fibrous mat 100 comprises an open network 115 of at least one polymer-based nanofiber 110. The diameter of the at least one nanofiber 110, also referred to as fiber, is ranging between 50 nm and 1500 nm.

The open network of nanofibers allows to distribute the contents of the metabolome sample within the fibrous mat and to efficiently adsorb and/or absorb the metabolome sample to/in the open network of the at least one nanofiber.

In embodiments of the present invention a cover layer 125 is provided at a top and/or at a bottom of the open network 115 of at least one nanofiber to exclude particles (e.g. bioparticles), with a size larger than 2000 Dalton, from interacting with the open network of nanofiber(s), by blocking these particles. 1 Dalton is equal to approximately 1,66×10⁻²⁷ Kg.

The cover layer 125 should be such that metabolites can pass through the cover layer to the open network of at least one nanofiber. Such a layer may for example be a PAN cover layer.

In embodiments of the present invention the cover layer may for example exclude the following bioparticles (this list is not exhaustive): bacteria, viruses, macromolecules such as DNA, RNA and/or proteins from interacting with the open network of at least one nanofiber.

In embodiments of the present invention the cover layer 125 may mainly (or even only) comprise a polymer that serves as a molecular weight cut-off filter and blocks the particles (e.g. bioparticles). In embodiments of the present invention the at least one polymer-based nanofiber of the open network, on the other hand, is partly hydrophobic and partly hydrophilic. It may comprise a blend of hydrophilic and hydrophobic moieties.

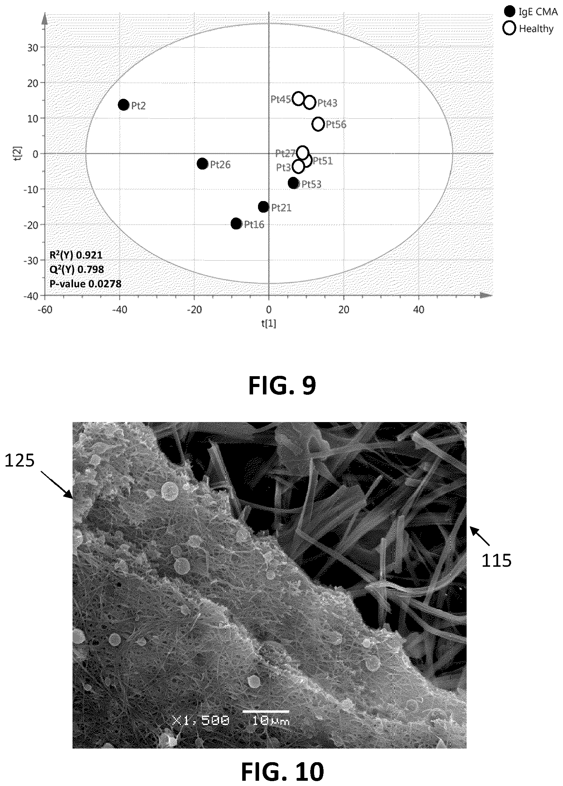

FIG. 10 shows a SEM image of a part of a fibrous mat, in accordance with embodiments of the present invention. The fibrous mat comprises an open network 115 of at least one polymer-based nanofiber and a cover layer 125 at one side of the fibrous mat. In this example the cover layer 125 is a PAN layer, and the open network 115 is made of one or more PVP/PS fibers.

In embodiments of the present invention the open network may comprise one nanofiber or a plurality of nanofibers. The weight% of the single nanofiber or of the plurality of nanofibers may at least be 50%. In embodiments of the present invention the weight% of the nanofiber(s) may be even more than 60%, or even more than 70%, or even more than 80%, or even more than 90% or even up to 100%

In embodiments of the present invention the hydrophilic/hydrophobic moiety ratio of the at least one nanofiber is selected such that deformation following exposure to water is less than 5% in size.

It is an advantage of embodiments of the present invention that a non-water soluble fibrous mat is obtained of at least one water soluble polymer by spinning it simultaneously with at least one non-water soluble polymer(s). Thus, a hydrophilic/hydrophobic moiety ratio can be obtained. This may also be achieved by using a single polymer which contains both the hydrophilic and hydrophobic moieties.

It is an advantage of embodiments of the present invention that, by introducing at least 50 weight% polymer-based nanofiber, and by selecting a hydrophilic/hydrophobic surface ratio of the at least one nanofiber, a fibrous mat can be obtained which can absorb water, and at the same time does not significantly deform.

The deformation may even be less than 3% in size, or even less than 1% in size. The deformation may be caused by swelling because of absorption of the water, or it may be caused by dissolving of some of the nanofibers. The deformation is measured along a chracterisitic dimension parallel with the surface of the mat. Only the coherent part of the mat is measured. The dissolving edges are not measured. Examples of deformation in size are given at the end of the description.

In embodiments of the present invention the hydrophilic/hydrophobic moiety ratio is between 10/90 and 75/25. The hydrophilic/hydrophobic moiety ratio may be between 20/80 and 75/25, or even between 30/70 and 70/30, or even between 40/60 and 60/40.

By providing the nanofiber(s) which is (are) partly hydrophobic and partly hydrophilic and/or by providing nanofibers of which some are hydrophobic and some are hydrophilic, a fibrous mat can be obtained which has an extraction coverage of metabolites including compounds bearing a broad range of polarities.

In embodiments of the present invention a hydrophilic nanofiber may be spun together with a hydrophobic nanofiber.

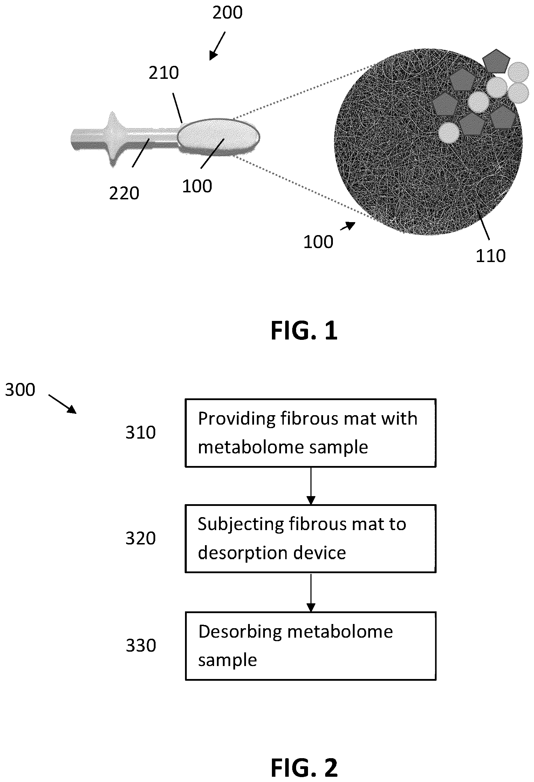

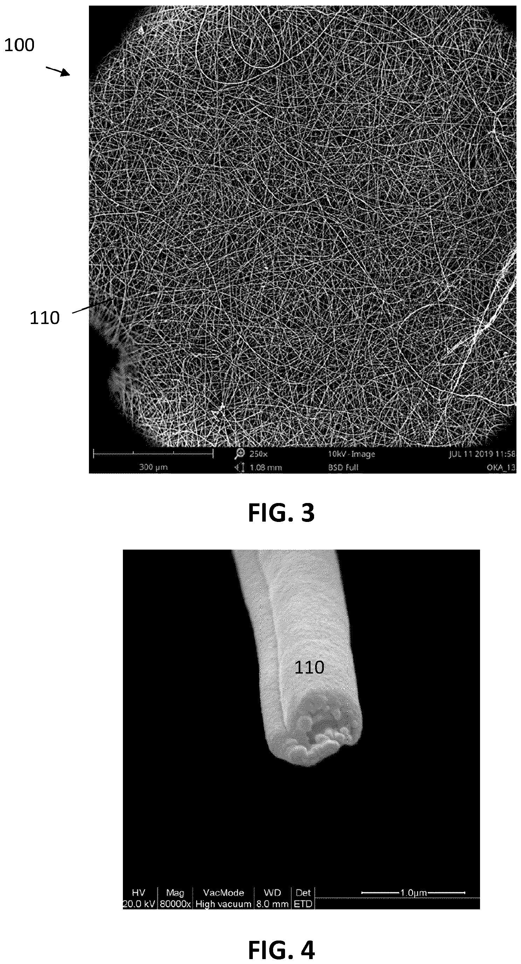

In embodiments of the present invention the fibrous mat may be obtained by electrospinning. An example of an open network of at least one polymer-based nanofiber of a fibrous mat, in accordance with embodiments of the present invention, is schematically illustrated in FIG. 1 and FIG. 3. FIG. 3 and FIG. 4 show scanning electron microscopy images (SEM) with different magnifications. FIG. 3 indicates homogeneous coverage of nanofibers. In the example, the average fiber diameter was 450 nm measured using a nanofiber diameter characterization tool (ImageJ). The samples were spun using polymer mixtures composed of PVP and PS (ranging from 35 to 55 weight% of PVP) and gave fiber diameters in the range of 300-800 nm.

It is an advantage of embodiments of the present invention that analytes and/or components including compounds can be efficiently captured by the fibrous mat. Adhesion is not only possible at the surface of the open network of at least one polymer based-nanofiber but also internally in the open network. This is enabled because the fibrous network is a fibrous open network, meaning that pores are present in the network. Using such a fibrous mat a physico-chemical broad range of molecules from the metabolome can be captured. The mass range (m/z) of analytes and/or components including compounds may for example be between 55 and 2000 Da. Their log P may range between -10 and 23.

| STANDARD | Compound class (HMDB/LIPID MAPS: class + subclass) | Log P (PubChem) | Monoistopic mass (HMDB) in Da |
|---|---|---|---|
| Taurolithocholate 3-sulfate | Steroids and derivates - bile acids, alcohols and derivates | 4.5 | 563.2587 |
| myo-inositol | Organooxygen compounds - alcohols and polyols | -3.7 | 180.0634 |
| D-Xylitol | Organooxygen compounds - carbohydrates and carbohydrate conjugates | -2.5 | 152.0684 |
| D-Mannitol | Organooxygen compounds - carbohydrates and carbohydrate conjugates | -3.1 | 182.0790 |
| DL-Lactic acid | Hydroxyacids and derivates- alpha hydroxy acids | -0.7 | 900.317 |
| Cortisone | steroids and derivates - hydroxysteroids | 1.5 | 360.1937 |
| 3-hydroxybutyric acid | hydroxy acids and derivates - bèta hydroxy acids | -0.5 | 104.0473 |
| Linoleic acid | Fatty acyls - linoleic acids and derivates | 6.8 | 280.2402 |
| L-carnosine | peptidomimetics | -4.0 | 226.1057 |
| Kynurenine | organo oxygen compounds - carbonyl compounds | -2.2 | 208.0848 |
| 1,2-Diheptadecanoyl-sn-glycero-3-phosphocholine | Glycerophospholipids - Glycerophosphocholines | 14.5 | 761.5935 |
| 1-Oleoyl-rac-glycerol (MG(18:1(9Z)/0:0/0:0)) | Glycerides | 6.9 | 356.2927 |
| Tricosanoyl sphingomyelin d 18:1/23:0 | sphingolipids - phosphosphingolipids | 16.1 | 798.6251 |
| L-cysteine | Carboxylic acids and derivates - Amino acids, peptides, and analogues | -2.5 | 121.0197 |
| L-arginine | Carboxylic acids and derivates - AA | -4.2 | 174.1117 |

A list of example analytes spiked in biofluids on a fibrous mat is shown in the table above. Their standard name can be found in the first column. Their compound class is obtained from the human metabolome database (HMDB) and from LIPD MAPS^{®}. As can be retrieved in HMDB they are all detectable in feces. Their Log P is retrieved from the PubChem database at the National Institutes of Health. Their monoisotropic mass is retrieved from HMDB. These analytes can be detected using a method in accordance with embodiments of the present invention, illustrating the physico-chemically broad coverage of a fibrous mat in accordance with embodiments of the present invention.

It is especially advantageous that not only the fibrous mat is particularly suitable for capturing the said compounds, but that it also allows a good desorption, and possibly also a good ionization of the metabolome. Ionization of the sample may for example be achieved by applying a voltage over the fibrous mat or sample, or by laser-assisted ionization, or by other known methods suitable for ionization.

In embodiments of the present invention the fibrous mat may be used for diagnosing a metabolome by taking the metabolome sample, by ionizing the metabolome sample and by analyzing the obtained ions to obtain a diagnosis for curative purposes. Analysis may for example be done by mass spectroscopy. Thereby the metabolites of the metabolome sample may be profiled and/or quantified.

In embodiments of the present invention the open network is non-woven. The at least one nanofiber may be biocompatible. The cover layer in particular may be biocompatible as well.

The diameter of the at least one nanofiber may range between 50 and 1500, or even between 50 and 1200, or even between 50 and 1000 nm, or even between 50 and 500 nm, or even between 400 nm and 800 nm, such as 200 nm, or 300 nm.

A fibrous mat 100 according to embodiments of the present invention may be used in laser-assisted ionization of the metabolome sample.

It is an advantage of a fibrous mat according to embodiments of the present invention that it allows good laser dissipation and subsequent desorption of captured analytes and/or components including compounds. Without being bound by theory it is assumed that the properties of the fibrous mat allow fast energy transfer and the absorption of laser energy. It is an advantage of embodiments of the present invention that the fibrous mat enables high transfer efficiency of the laser energy for the analysis of biological samples.

It is advantageous to use a fibrous mat according to embodiments of the present invention for taking a metabolome sample and for laser-assisted ionization of the metabolome sample because it avoids the use of an organic matrix (deemed especially exquisite for the analysis of small molecules) and substantially lessens the issue of matrix effects, yet reduces the time-consuming sample pretreatment and analysis to a fraction of a minute. Water molecules, which are present to a large extent in biofluids such as e.g. faeces, urine, saliva and blood, can act as an endogenous matrix since the IR laser wavelength may be tuned (e.g. 2.94 µm) to excite the most intense vibrational band (O-H stretching mode) of water molecules.

In embodiments of the present invention the open network comprises pores. The pores are formed between the nanofibers. These pores are part of the interconnected nanofibrous network. The interconnected pores allow to distribute the contents of the metabolome sample within the fibrous mat and to efficiently adsorb (and subsequently, upon instrumental analysis, desorb) the sample to the surface of the nanofibers.

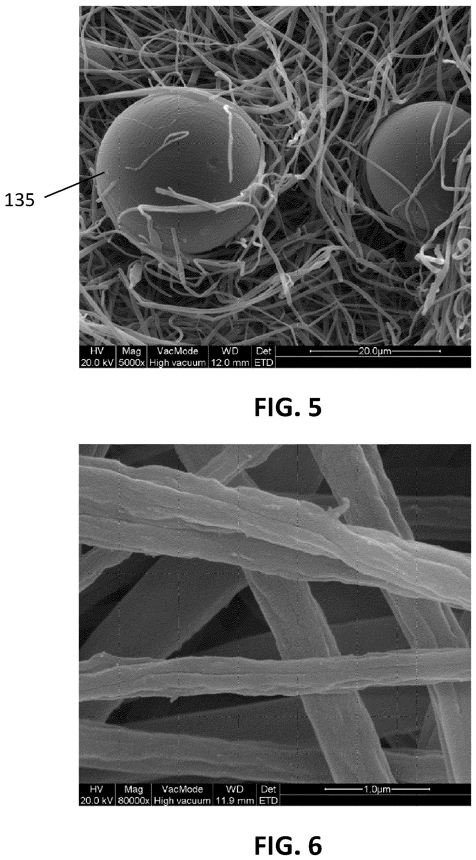

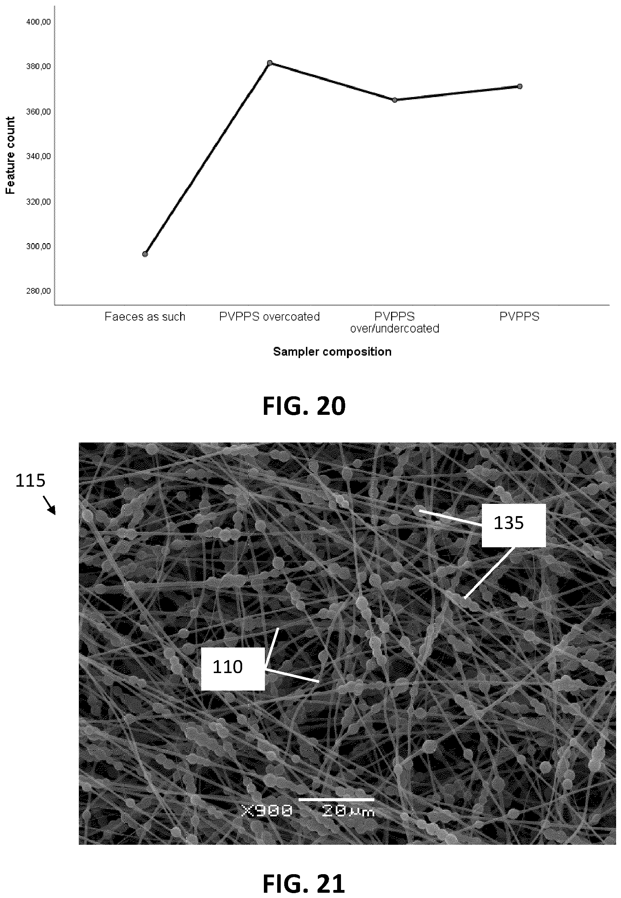

In embodiments of the present invention the fibrous mat may comprise nano- or microparticles 135 which are incorporated in the interconnected network. Examples thereof are shown in FIG. 5 and FIG. 21. These figures show SEM images of a fibrous entangled open network of a fibrous mat, in accordance with embodiments of the present invention, in which microparticles are embedded. For this example, the microparticles were blended into the polymer solution consisting of PVP/PS. These nano- or micro-particles may be (co-)polymers which may have various structures. They may for example be spherical or they may have a tubular structure. They may for example be carbon nanotubes. Their size (e.g. internal diameter for spheres, length for tubes) may for example range between 100 nm and 1500 nm or between 1 µm and 40 µm, for example up to 30 µm, or up to 20 µm, or up to 10 µm.

In embodiments of the present invention the nano- or micro-particles may be coated. They may for example be polar coated such that they attract more the hydrophilic molecules.

It is an advantage of embodiments of the present invention that the metabolome coverage can be increased by incorporating nano- or micro-particles in the interconnected network. Moreover, they allow a good dissipation of laser energy, which is advantageous for laser-assisted ionization of the metabolome sample, for example.

FIG. 6 shows a SEM image of one, a PVP/PS phase hydrophilic/hydrophobic phase-separation, of the different morphologies the electrospinning process can deliver, i.e. from intensely blended to phase-separated materials in covering the holistic metabolome. In this example the fibrous mats were spun using polymer mixtures composed of PVP and PS (at 55 weight% of PVP) and gave fiber diameters in the range of 300-800 nm.

A sampling device 200 according to embodiments of the present invention, e.g. swab, may be formed by securing the fibrous mat to a carrier 210. An example thereof is illustrated in FIG. 1. The fibrous mat may envelop the carrier 210. It is thereby advantageous that the fibrous mat forms one and the same enveloping phase with respect to the surface of the solid carrier. In embodiments of the present invention the fibrous mat is secured to an outer end of the sampling device. This outer end is also referred to as the swab head. The swab head may for example be attached to a stainless steel handle 220. The invention is not limited thereto. Also other materials, preferably non-flammable materials can be considered as well. These may for example be thermally resistant plastics such as polypropylene or polyethylene. A sampling device, according to embodiments of the present invention may allow sampling at ambient conditions. It may be configured as a medical swab for rectal usage capable of capturing a broad, physico-chemically diverse range of metabolites and directly applicable as a substrate for ambient SALDI at the surface of the fibrous mat.

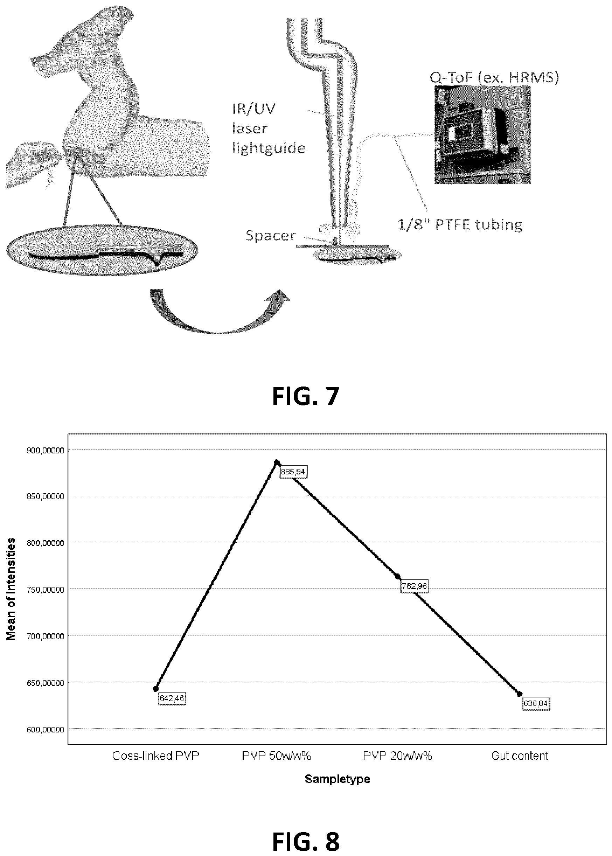

In a second aspect, embodiments of the present invention relate to a method 300 for desorption of a metabolome sample. A flow chart of such a method is illustrated in FIG. 7. The method comprises providing 310 a fibrous mat which carries a metabolome sample. The fibrous mat comprises (a) an open network of at least one polymer-based nanofiber, and the diameter of the at least one nanofiber is ranging between 50 nm and 1500 nm and (b) a cover layer is provided at a top and/or at a bottom of the open network of at least one nanofiber to exclude particles with a size larger than 2000 Da from interacting with the open network of at least one nanofiber by blocking and/or excluding access to these particles.

The method, moreover, comprises subjecting 320 the fibrous mat to a device configured for desorbing (or desorbing and ionizing) the metabolome sample and desorbing 330 the metabolome sample. Desorption of the metabolome sample may be combined with ionization of the metabolome sample which may be done by laser-assisted ionization.

In embodiments of the present invention the cover layer may be cleaned by washing (e.g. a quick wash of 10 s) before subjecting the fibrous mat to the device configured for desorbing (and ionizing) the metabolome sample. This wash may be optimized per biofluid. It is an advantage of embodiments of the present invention that particles (e.g. bioparticles) are removed prior to analysis while the metabolites which are absorbed and/or adsorbed to/in the open network of at least one polymer-based nanofiber (e.g. PVP/PS fibers), are not removed by the washing.

The generated plume of analytes and/or components including compounds, resulting from the ionization of the metabolome sample, may be introduced in an MS inlet (e.g. for HRMS analysis). The prior art separation step, which is conventionally performed by means of LC to reduce matrix effects, is thus replaced by the usage of a very efficient sampling and compound enrichment strategy by using a fibrous mat according to embodiments of the present invention for taking the metabolome sample and for ionizing the metabolome sample directly from the fibrous mat 100.

In embodiments of the present invention providing 310 the fibrous mat comprises taking the metabolome sample using the fibrous mat. A fibrous mat according to embodiments of the present invention allows to capture analytes and/or components including compounds from the biological matrix of interest, e.g. the rectum or mouth. The fibrous mat may be designed for broadscale capturing of analytes and/or components including compounds or for targeted capturing of compounds.

The fibrous mat 100 may be placed for a required amount of time, during which the maximal extraction is performed, in the location where the sample is taken. It may, for example, be positioned in the rectum (1 à 2 cm past the anal verge) and rotated gently 360°. Subsequently, when the extraction, such as microextraction, has occurred to a sufficient degree (e.g. approximately in the minute range) the fibrous mat is removed and placed in a sample holder. During clinical research the fibrous mat may be placed into a sterilized storage container for transport to a laboratory. The sample holder may be a rotating sample holder.

The sample holder may be part of a set-up configured for ionization of the metabolome sample. This may be a laser set-up for carrying out desorption of the analytes and/or components including compounds extracted in the fibrous mat. The obtained gas-phase analytes and/or components including compounds may be transported through a tubing (e.g. PTFE tubing) for further analysis. The tubing may be positioned just above the sample holder and directly connected to the inlet of the analysis equipment (e.g. the MS instrument). An example thereof is illustrated in FIG. 7. PTFE is a versatile plastic characterized by high chemical and thermal resistance and is very non-reactive. For example, a laser desorption ionization (LDI) set-up may be hyphenated with the HRMS apparatus through a PTFE tube allowing the transfer of the generated gas-phase ions into the MS inlet, thereby enabling remote analysis and freedom of movement.

Use of a fibrous mat according to embodiments of the present invention for taking a metabolome sample and subsequent ionization of the metabolome sample may for example allow to ultimately screen for predictive and prognostic indicators pointing towards metabolic disturbance during early childhood to prevent and/or predict the prognosis of the development of metabolic diseases and long-term complications. Easy, in situ and in vivo rectal or oral sampling is enabled using a biocompatible and flexible nanofibrous electrospun polymeric composite fibrous mat in accordance with embodiments of the present invention.

It is an advantage of embodiments of the present invention that using the fibrous mat 100 as a substrate for a metabolome sample in a SALDI-HRMS approach opens doors towards transcending the current state of the art of metabolomics for precision medicine. The application empowers tackling a number of important (pediatric) health care issues such as obesity/(pre)diabetes predication and preventions as well as cow's milk allergy diagnosis, for instance.

A fibrous mat 100, according to embodiments of the present invention may comprise specific components (polymers, particles) aiming at a broad metabolome coverage. A fibrous mat according to embodiments of the present invention may be partly hydrophobic and partly hydrophilic. Depending on the application the percentage hydrophobic surface/hydrophilic surface may vary. For example, when the application aims to have the maximum stool metabolome coverage a hydrophobic/hydrophilic ratio of 30-60% / 40-70%, for example approximately 40% / 60% is preferable. Depending on the application other ratios ranging from purely hydrophilic to purely hydrophobic may be used.

The mixed hydrophobic/hydrophilic character of the fibrous mat may be obtained through various implementations. For example at least one nanofiber may be partly hydrophobic and partly hydrophilic and/or at least one nanofiber may be hydrophobic and at least one nanofiber may be hydrophilic and/or the core of a nanofiber may be hydrophilic and the shell hydrophobic or vice versa and/or nano-, or micro-particles of the fibrous mat may be hydrophobic or hydrophilic. In embodiments of the present invention one fiber may comprise a hydrophilic component and a hydrophobic component. In embodiments of the present invention hydrophilic and hydrophobic material may be added to the at least one fiber.

A fibrous mat according to embodiments of the present invention may comprise a mixture of polymers. These may be hydrophilic, hydrophobic, or they may even be polymers containing both hydrophilic and hydrophobic moieties such as co-polymers. Thus, a fibrous mat which has an extraction coverage of analytes and/or components including compounds bearing a broad range of polarities can be obtained.

Examples of hydrophilic polymers are: polyacrylate (PA), polyacrylonitrile (PAN), polyvinylpyrrolidone (PVP), polyamide, crosslinked PVP.

Examples of hydrophobic polymers are: polystyrene (PS), divinylbenzene (DVB) such as for example polydivinylbenzene (PDVB), polydimethylsiloxane (PDMS), sulfonated tetrafluoroethylene (protonating capacities).

Examples of hydrophilic/hydrophobic co-polymers: PDVB-co-PVP, hydrophilic-DVB.

The at least one nanofiber may for example comprise polystyrene and polyvinylpyrrolidone. The PVP/PS weight ratio (%) may for example range from 70/30 to 10/90, or even between 60/40 and 40/60.

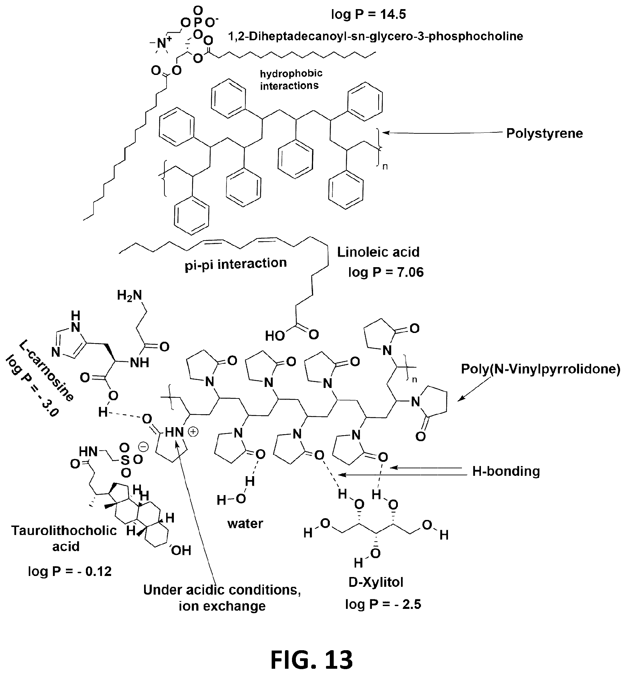

Interactions of nanofiber moieties with analytes from the table above, are illustrated using the chemical drawing in FIG. 13. In this example the microfiber comprises PVP/PS. The following type of interactions are shown: H-bonding, ionic interactions (cation exchange, anion exchange), hydrophobic interactions (π-π intercations), Vander Waal's interactions (dipole-dipole, ion-dipole, London forces). This figure illustrates that the coverage can be broadened by combining hydrophobic and hydrophilic moieties in one nanofiber. It is an advantage of some embodiments of the present invention that the fibrous mat does not deform even though hydrophilic moieties are present in the fibrous mat. This is achieved by providing a fibrous mat which comprises at least one nanofiber having a suitable combination of hydrophilic/hydrophobic moieties.

In embodiments of the present invention chemical interactions and physical interactions through the use of the combination of hydrophilic and hydrophobic polymers in a (nano)fibrous mat may apply and benefit from the (nano)fibrous mat's high specific surface area. Chemical interactions (pi-pi stacking, Van Der Waals interactions, H-bonding, covalent binding, etc.) impart adsorption and absorption and include both chemical and physical binding and physical adhesion.

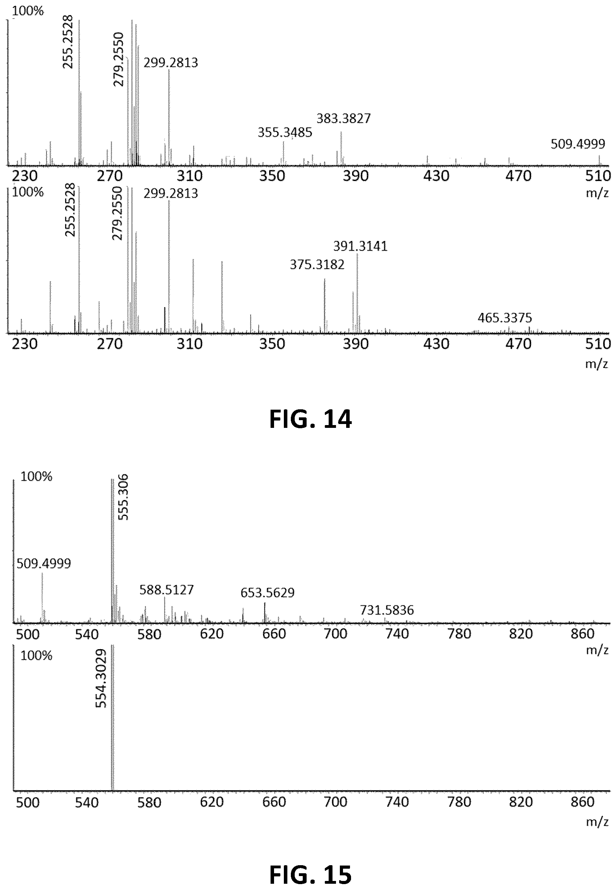

FIG. 14 show the mass spectra of faeces obtained using a fibrous mat and without using a fibrous mat. The spectra are obtained for a mass range from 200 to 520 Dalton.

The top spectrum shows a spectrum obtained using a method in according with embodiments of the present invention (i.e. by collecting a metabolome sample, feces for example, with the fibrous mat, by desorbing the metabolome sample, and by ionizing the metabolome sample and by collecting the spectrum).

The bottom spectrum shows the spectrum of the biofluid itself (without using a fibrous mat).

Similarly as in FIG. 14, the top spectrum in FIG. 15 is obtained using a fibrous mat, in accordance with embodiments of the present invention, and the bottom spectrum shows the spectrum of the biofluid, feces for example, itself. The spectra are obtained for a mass range from 480 to 880 Dalton.

As can be seen from these graphs a broad coverage is obtained when using a fibrous mat in accordance with embodiments of the present invention.

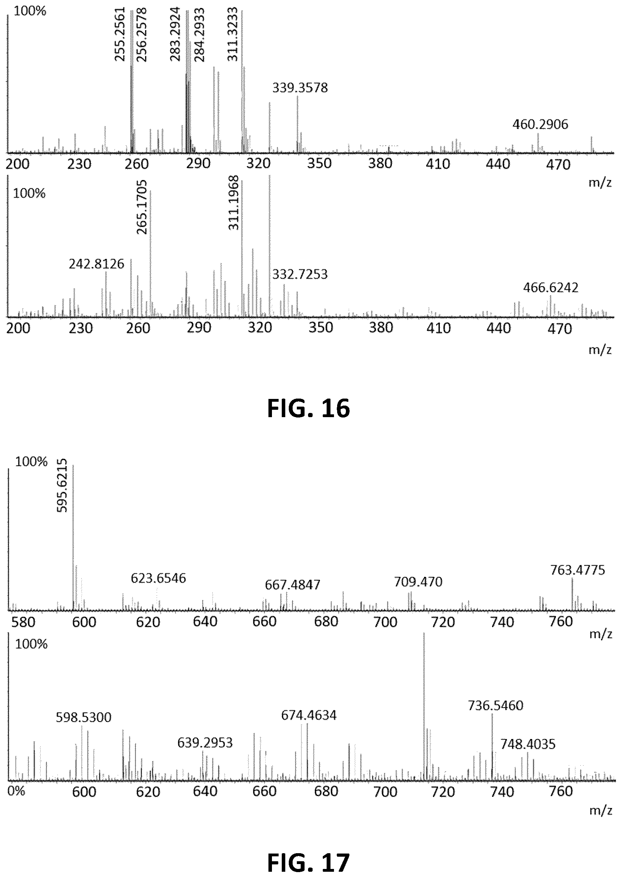

Similarly as FIG. 14 and FIG. 15, FIG. 16 and FIG. 17 show the spectra of saliva obtained using a fibrous mat (above) and without using a fibrous mat (below). For FIG. 16 the spectra are obtained from 200 to 500 Dalton. For FIG. 17 the spectra are obtained for a mass range from 570 to 780 Dalton.

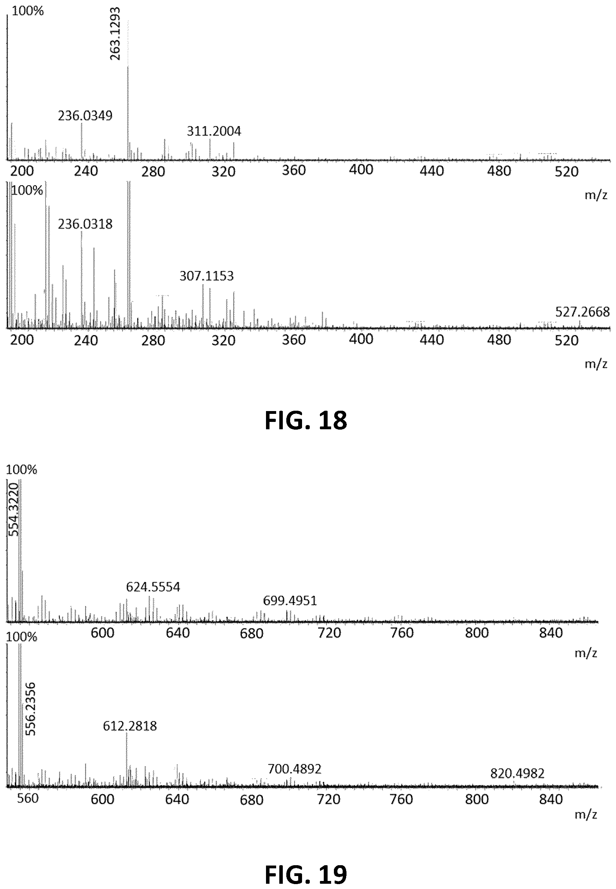

FIG. 18 and FIG. 19 show the spectra of urine obtained using a fibrous mat (above) and without using a fibrous mat (below). For FIG. 18 the spectra are obtained for a mass range from 200 to 550 Dalton. For FIG. 19 the spectra are obtained for a mass range from 550 to 870 Dalton.

FIG. 20 shows the feature count for different sampler (i.e. a fibrous mat) compositions. On the horizontal axis the following compositions are present (from left to right): Faeces as such, a PVP/PS fibrous mat which is overcoated, a PVP/PS fibrous mat which is over- and undercoated with a cover layer (for example PAN), a PVP/PS fibrous mat. The open network is obtained by solving polymer components in a solvent, and by electrospinning at least one polymer. The PVP/PS ratio is 60% versus 40% in weight. The polymer components are solved in CHCl₃:DMF (2:1). The cover layer may be obtained by solving PAN in dimethylformamide (e.g.: 5% (w/v) in DMF), and by, for example, electrospinning.

The vertical axis shows the feature count. The feature count can for example be obtained from the mass spectra. The feature count is, in this experiment, obtained for a mass range from 600 to 800 Dalton. As can be seen from this figure the differences between with or without coating are marginal.

Specific moieties such as hydrophilic divinylbenzene (DVB, N-methyl-N-vinylamide) and Waters Oasis HLB^{®} may be added to the fibrous mat. By selecting the polymers and the moieties the extraction coverage of analytes and/or components including compounds bearing a broad range of polarities may be optimized (ranging from amino acids, i.e. polar metabolites, to triacylglycerols, i.e. non-polar metabolites).

Optimization may be done in design of experiment (DOE) and response surface modelling (RSM) approaches (e.g. JMP 14 (SAS) Software). The DOE and RSM may thereby focus on maximizing the metabolome coverage and comparing the signal intensities gained upon SALDI of the impregnated fibrous mat versus the analysis of biofluids as such.

The polymers from the list are characterized with features necessary towards using the fibrous mat for taking a metabolome sample, i.e., biocompatibility, flexibility, elasticity and stability throughout a broad pH range (e.g. target mean intrarectal pH of children is 6.75 but can go as high as 13).

In embodiments of the present invention the polymers may be selected for their mechanical stability and thermoplastic behavior (e.g. withstanding the laser irradiation temperature).

It is an advantage of embodiments of the present invention that the extraction phase is a homogeneous fibrous mat 100 with an extraction coverage of analytes and/or components including compounds bearing a broad range of polarities (log P may range between -10 and 23). This mat can be applied to a carrier 210. It is an advantage of embodiments of the present invention that in one go a comprehensive coverage can be obtained.

FIG. 8 shows a graph illustrating the metabolome coverage of different fibrous mats in accordance with embodiments of the present invention compared to feces as such. This graph shows a statistical (untargeted) analysis based on the evaluation of total ion current (TIC) repeated measurements by the electrospun polymers mentioned (crosslinked PVP, PVP 50% - PS 50% and PVP 20% -PS 80%). In this analysis, it was shown that the electrospun fibrous mats showed similar (not significantly different) and significantly higher coverage (in terms of TIC) when compared to the analysis of feces as such (i.e. not impregnated on electrospun fibrous mats) by means of the said LDI technique.

FIG. 9 shows a multivariate OPLS-DA model from stool of cow's milk allergy (CMA) children on a fibrous mat, in accordance with embodiments of the present invention, enabling a valid classification according to health state. The full black circles indicate IgE CMA. The white circles with black circumference indicate healthy patients.

In embodiments of the present invention the material of the fibrous mat may be selected to secure an improved desorption, and/or a decreased and/or a clean background signal upon SALDI. Optimization may be done by a DOE which may include the distance between the fibrous mat and the laser which is used for ionizing 330 the metabolome sample (a rotating sample holder may be used for complete analysis of the surface of the fibrous mat), the rotation of the fibrous mat (° and rpm), the pulse energy (mJ), the pulse width (ns) and the ablation time (s).

When measuring using SALDI-REIMS-MS, the SALDI-REIMS-MS parameters may be finetuned for both polarity modalities considering the biological matrix.

A fibrous mat 100 according to embodiments of the present invention may for example be used for taking a metabolome sample for many common human diseases of which the origin has been associated with disturbances in gastrointestinal microbial composition, such as inflammatory bowel disease (IBD), colorectal cancer, allergies (e.g. cow's milk allergy), obesity, diabetes mellitus (DM), etc. Regarding obesity for instance, there exists an ever-increasing emergence of overweight, obesity and related metabolic perturbations worldwide in adults, adolescents and even alarmingly rising during early childhood. As such, screening the young population as soon as possible is highly needed. Childhood obesity is associated, amongst others, with a condition of marked insulin resistance. To date, there still is a lack of international reference values for '(ab)normal' insulin sensitivity. Next to childhood obesity, the increasing prevalence of food allergies in children, such as cow's milk allergy, is of major concern, as currently the only treatment for this disease is the implementation of an elimination diet. The latter highlights the importance of an accurate and specific diagnosis. A method 300 for ionization of a metabolome sample, according to embodiments of the present, allows to obtain an ion stream which may be diagnosed in accordance with embodiments of the present invention. State of the art methodologies may be used for diagnosing the ion stream. A fibrous mat 100 according to embodiments of the present invention allows taking the metabolome sample and can be used in laser assisted ionization of the metabolome sample. It, therefore, enables to correlate mass spectra with molecular signs of metabolic disturbance and to provide insights into disease markers and aberrant biological pathways to improve (personalized) intervention strategies such as therapeutic approaches. This is illustrated in FIG. 2 which illustrates taking the metabolome sample using the fibrous mat and desorbing the sample. Desorption of the sample may be combined with ionization of the sample. This may for example be achieved by laser desorption and ionization of the sample on the fibrous mat. The ionized sample can for example be analyzed using ion mobility spectrometry.

It is an advantage of embodiments of the present invention that it requires a lower amount of the biological sample than in case a whole biofluid sample needs to be collected. Moreover, swabbing can be performed at any time. The fibrous mat, according to the present invention, allows to efficiently capture the compound(s) and, moreover, allows a good laser (energy) dissipation and subsequent desorption of captured analytes and/or components including compounds.

Surface assisted laser desorption ionization (SALDI) hyphenated to HRMS, may for example be used as analytical methodology. It is an advantage of embodiments of the present invention that this eliminates long sample preparation and analysis times as a reduction in time of approximately 70% compared to standard LC-MS acquisition (averaged at 15 minutes) can be achieved without even accounting for the sample preparation procedure(s) required for such analytical platform. Using a fibrous mat according to embodiments of the present invention for taking the metabolome sample and for laser assisted ionization of the metabolome sample, moreover, greatly lowers the use of expensive, environmental (toxic) hazardous (organic) reagents. When using a fibrous mat in accordance with embodiments of the present invention specific moieties and a varying polymer mixture composition for an improved extraction coverage of analytes and/or components including compounds bearing a broad range of polarities may be applied. The microextraction principle using the fibrous mat, according to embodiments of the present invention, further minimizes the contamination of instrumentation (e.g. an MS instrument) and deals with possible matrix interferences due to analysis of the crude biological sample extracted onto the fibrous mat.

In embodiments of the present invention the ions may be formed outside the eventual measurement instrument which may for example be a mass spectrometer. As discussed earlier the ionization 330 of the metabolome sample on the fibrous mat may be achieved using a laser.

Ambient ionization MS (AIMS) approaches may be applied for analyzing the metabolome sample on the tissue. Rapid evaporative ionization MS (REIMS) is one of the ambient ionization MS (AIMS) approaches.

A fibrous mat, according to embodiments of the present invention may be obtained by electrospinning. The fibrous mat may for example be made by:
- solving at least on polymer component in a solvent wherein hydrophobic and hydrophilic moieties are present in the at least one polymer component,
- electrospinning of the at least one dissolved polymer component thereby forming an open network of at least one polymer-based nanofiber, wherein the at least one nanofiber is spun with a diameter ranging between 50 nm and 1500 nm,
- providing a cover layer at a top and/or at a bottom of the open network of at least one nanofiber to exclude particles with a size larger than 2000 Da from interacting with the open network of nanofiber(s) by blocking and/or excluding access to these particles.

In embodiments of the preset invention more than one polymer component may be dissolved. One may for example be hydrophobic and one hydrophilic. In some embodiments of the present invention a single polymer component may comprise hydrophobic and hydrophilic moieties.

The cover layer may be applied using electrospinning. Alternatively, dip coating, sputtering, spin coating, etc. may be used for applying the cover layer. The electrospinning technique is especially suitable for creating fibers with a diameter in the nano range. In embodiments of the present invention the fibrous mat is created by one or more fibers which are entangled.

Hydrophobic and a hydrophilic components may be dissolved in the solvent for electrospinning. Thus, a nanofiber can be obtained that is partly hydrophobic and partly hydrophilic.

The electrospinning may be done on a carrier 210. Thus, a sampling device can be formed by directly spinning the fibrous mat 100 on the carrier 210.

In embodiments of the present invention biocompatible polymers are used for the fibrous mat. Biocompatibility is thereby considered as not causing toxic reactions or immunological rejection. A biocompatible coating may be applied to the fiber. The PAN is for example a biocompatible coating (or cover layer). The biocompatible coating endows the device to be directly introduced into complex biological matrices. It is an advantage of embodiments of the present invention that a fibrous mat comprising an open network of at least one polymer-based nanofiber is able to capture the analytes and/or components including compounds of interest, assisted by its high porosity and large surface area of the nanofibers enabling substantial compound loading capacities. Additionally, the high surface area allows fast energy transfer and the absorption of laser energy. The conductive nanofibrous polymeric biocompatible mat enables a good transfer efficiency of the laser energy for the analysis of biological samples.

Molecular imprinting of the polymers (MIP) may be used to increase the extraction efficiency and to contribute to the sensitivity and specificity of the analysis by further reducing possible matrix effects.

The table below shows different experiments in a fractional factorial design wherein different fibrous mats are used for taking a faeces or rectal sample and for desorbing and obtaining the number of molecular features of the sample. Thus, the PVP/PS mixture can be optimized for faeces or rectal content. In each experiment a different fibrous mat is used. The first column shows the experiment number. The second column shows the spinning duration of the PVP/PS nanofibers. The third column shows the duration for applying the PAN cover layer. The fourth column shows the weight% of PVP to the total mass of polymer. The fifth column shows the total polymer weight% in solution. The sixth column shows the number of molecular features detected. The seventh column shows the percentage of features with CV < 30%, and the last column shows the summarized normalized intensity. These are obtained based on minimum 3 repititions and averaged using the obtained mass spectra.

| Exp Name | Spinning duration (h) | PAN (min) | PVP (weight% to total mass of polymer) | Total polymer weight (weight% in solution) | Number molecular features | % Features with CV < 30% | Summarized normalized intensity |
|---|---|---|---|---|---|---|---|
| N1 | 3 | 30 | 20 | 8 | 1408 | 73.84 | 798.59 |
| N2 | 8 | 30 | 20 | 8 | 1347 | 75.37 | 808.04 |
| N3 | 3 | 120 | 20 | 8 | 1557 | 75.02 | 964.23 |
| N4 | 8 | 120 | 20 | 8 | 1354 | 66.25 | 724.68 |
| N5 | 3 | 30 | 60 | 8 | 1561 | 82.38 | 866.36 |
| N6 | 8 | 30 | 60 | 8 | 1492 | 57.24 | 838.58 |
| N7 | 3 | 120 | 60 | 8 | 1494 | 54.89 | 863.23 |
| N8 | 8 | 120 | 60 | 8 | 1436 | 76.01 | 740.14 |
| N9 | 3 | 30 | 20 | 12 | 1456 | 74.55 | 775.96 |
| N10 | 8 | 30 | 20 | 12 | 1350 | 65.73 | 798.69 |
| N11 | 3 | 120 | 20 | 12 | 1437 | 65.99 | 743.66 |
| N12 | 8 | 120 | 20 | 12 | 1412 | 71.67 | 802.68 |
| N13 | 3 | 30 | 60 | 12 | 1491 | 62.66 | 793.74 |
| N14 | 8 | 30 | 60 | 12 | 1508 | 75.23 | 769.76 |
| N15 | 3 | 120 | 60 | 12 | 1548 | 71.93 | 909.35 |
| N16 | 8 | 120 | 60 | 12 | 1448 | 62.52 | 840.58 |
| N17 | 5.5 | 75 | 40 | 10 | 1552 | 72.42 | 893.13 |
| N18 | 5.5 | 75 | 40 | 10 | 1463 | 60.85 | 842.04 |
| N19 | 5.5 | 75 | 40 | 10 | 1518 | 70.45 | 788.63 |

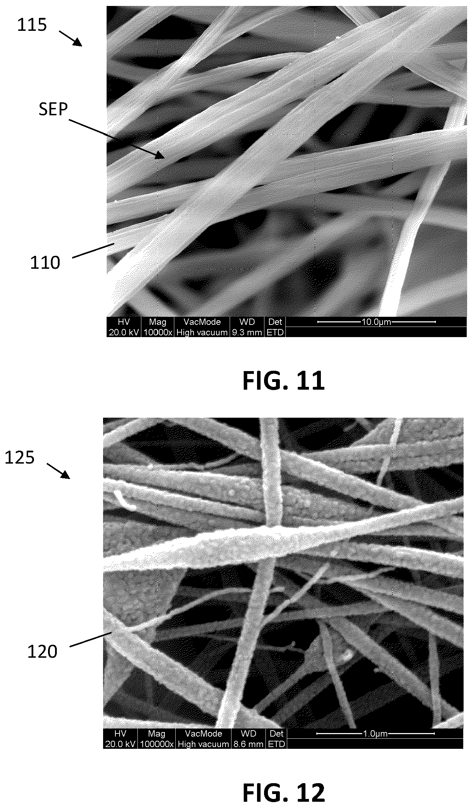

FIG. 11 shows a SEM Image of PVP/ PS nanofibers with most optimal action towards faeces molecular fingerprinting. In the SEM image the phase separation between PVP and PS is shown (this is indicated by the arrow SEP).

FIG. 12 shows a SEM image of PAN fibers 120 of a cover layer 125 of a fibrous mat, in accordance with embodiments of the present invention.

As discussed earlier, the fibrous mat may comprise nano-, or microparticles incorporated in the open network of the one or more nanofibers. SEM images of such an open network 115 comprising microparticles 135 are shown in FIG. 5 and FIG. 21. The fibrous mat is a mat comprising PS/PVP fibers. In the examples 3-9% of HLB particles (1.5-20 µm in size) where dispersed in 8 weight% PS/PVP solution. The solvent was DMF/CHCl₃. The fibers, obtained by electrospinning were below 900 nm in diameter. The images show particles that are trapped inside the nanofibers and in some cases are part of the nanofibers.

The table below shows different experiments in a fractional factorial design wherein different fibrous mats are used for taking a saliva sample and for desorbing and obtaining the number of molecular features of the sample. Thus, the PVP/PS mixture and PAN cover can be optimized for saliva.

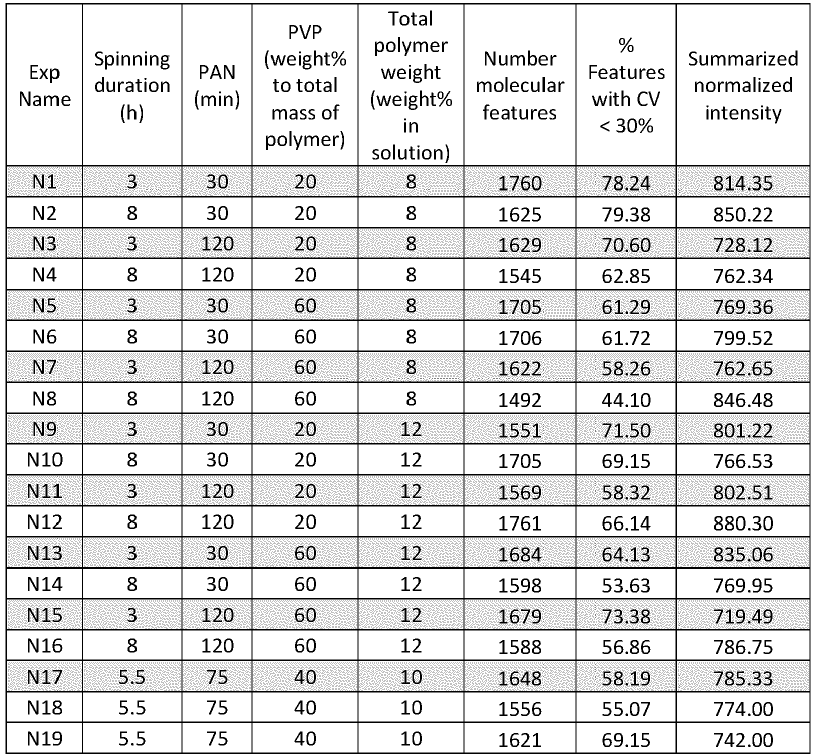

The table below shows different experiments in a fractional factorial design wherein different fibrous mats are used for taking a urine sample and for desorbing and obtaining the number of molecular features of the sample. Thus, the PVP/PS mixture and PAN cover can be optimized for urine.

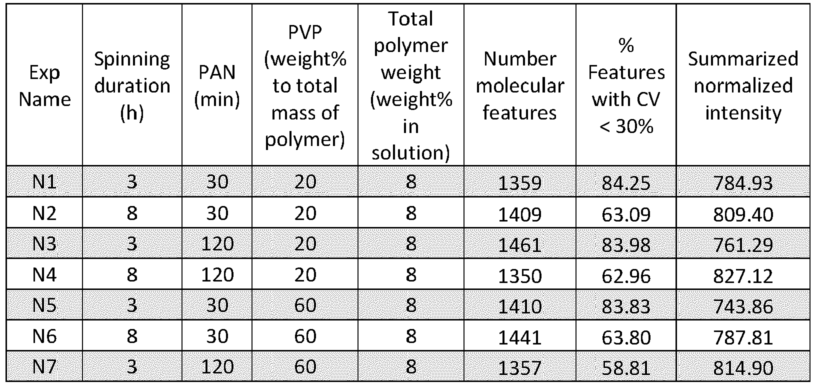

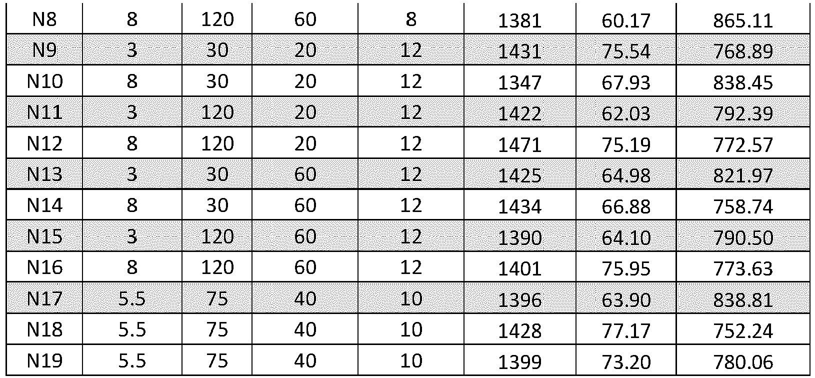

In the following paragraphs deformation experiments following water exposure are discussed. Two working membranes (i.e. fibrous mats) were selected with varying PVP/PS weight% (40/60-60/40) and a negative control membrane with PVP/PS weight% (80/20).

For the deformation test protocol the following steps were executed:
- Membranes were cut in 3x3 cm;
- Conditioned at 60°C for 48 hours;
- Membranes were submerged in water in petridish for 30 min, 1 and 2 hours;
- Membrane dimensions were measured again using a measuring scale.

The results for the N6 membrane are shown in the table below.

| Time (min) | Dimensions (cm) | | | Mean | SD | CV |
|---|---|---|---|---|---|---|
| | Replicate- 1 | Replicate- 2 | Replicate-3 | | | |
| 0 | 3 | 2.9 | 2.9 | 29.333 | 0.0577 | 19.682 |
| 1 | 3 | 2.9 | 2.9 | 29.333 | 0.0577 | 19.682 |
| 5 | 3 | 2.9 | 2.9 | 29.333 | 0.0577 | 19.682 |
| 15 | 3 | 2.9 | 2.9 | 29.333 | 0.0577 | 19.682 |
| 30 | 3 | 2.9 | 2.9 | 29.333 | 0.0577 | 19.682 |
| 60 | 3 | 2.9 | 2.9 | 29.333 | 0.0577 | 19.682 |
| 120 | 3 | 2.9 | 2.9 | 29.333 | 0.0577 | 19.682 |

The results for the N18 membrane are shown in the table below.

| Time (min) | Dimensions (cm) | | | Mean | SD | CV |
|---|---|---|---|---|---|---|
| | Replicate- 1 | Replicate- 2 | Replicate-3 | | | |
| 0 | 3 | 3 | 2.9 | 29.667 | 0.0577 | 19.461 |
| 1 | 3 | 3 | 2.9 | 29.667 | 0.0577 | 19.461 |
| 5 | 3 | 3 | 2.9 | 29.667 | 0.0577 | 19.461 |
| 15 | 3 | 3 | 2.9 | 29.667 | 0.0577 | 19.461 |
| 30 | 3 | 3 | 2.9 | 29.667 | 0.0577 | 19.461 |
| 60 | 3 | 3 | 2.9 | 29.667 | 0.0577 | 19.461 |
| 120 | 3 | 3 | 2.9 | 29.667 | 0.0577 | 19.461 |

The measurements were carried out in triplicate and were obtained using a measurement setup as illustrated in the pictures in FIG. 22. A ruler is present behind each fibrous mat which allows to measure a characteristic dimension (e.g. width or diameter) of the mat at different moments in time. It was observed that the membranes did not shrink, swell, nor dissolve in distilled water. Also no change in colour was observed.

For the negative control membrane with PVP/PS % of (80/20), an incoherence of the mat was observed. A loss of fibers was observed and deformation of the membrane was observed. Moreover, the membranes got stuck to to the petridish and when removed formed a gel like structure. FIG. 23 shows such an example illustrating nanofibers which are falling apart from the membrane and dissolving in distilled water. FIG. 24 shows how the membranes are sticking to the petri dish.

## Claims

1. A fibrous mat (100) configured
for use in taking a metabolome sample and for use in desorption of the metabolome sample, the fibrous mat (100) comprising an open network (115) of at least one polymer-based nanofiber (110), wherein the diameter of the at least one nanofiber (110) is ranging between 50 nm and 1500 nm,
**characterized in that** a cover layer (125) is provided at a top and/or at a bottom of the open network of at least one nanofiber to exclude particles with a mass larger than 3,32×10⁻²⁴Kg (2000 Da) from interacting with the open network (115) of at least one nanofiber (110) by blocking and/or excluding access to these particles.

2. A fibrous mat (100) according to claim 1 wherein a hydrophilic/hydrophobic moiety ratio of the at least one nanofiber (110) is selected such that deformation of the fibrous mat is less than 5% in size after exposure to water.

3. A fibrous mat (100) according to claim 2, wherein the hydrophilic/hydrophobic moiety ratio ranges from 75/25 to 10/90.

4. A fibrous mat (100) according to any of the previous claims configured for use in desorption and ionization of the metabolome sample.

5. A fibrous mat (100) according to any of the previous claims configured for use in laser-assisted desorption of the metabolome sample.

6. A fibrous mat (100) according to any of the previous claims configured for use in diagnosis of the metabolome sample.

7. A fibrous mat (100) according to any of the previous claims the fibrous mat comprising nano- or micro-particles (135) which are incorporated in the open network (115) of the at least one polymer-based nanofiber or in the at least one polymer-based nanofiber.

8. A fibrous mat (100) according to any of the previous claims wherein the at least one nanofiber (110) is comprising one or more materials selected from:
a list of hydrophilic polymers comprising polyacrylate, polyacrylonitrile, polyvinylpyrrolidone, and/or from a list of hydrophobic polymers comprising polystyrene, divinylbenzene, polydimethylsiloxane, polydivinylbenzene,
and/or from a list of combined hydrophobic/hydrophilic polymers comprising polydivinylbenzene-co-polyvinylpyrrolidone, hydrophilic- divinylbenzene, crosslinked polyvinylpyrrolidone.

9. A sampling device (200) comprising a fibrous mat (100) according to any of the previous claims and a carrier (210) to which the fibrous mat is secured.

10. A method (300) for desorption of a metabolome sample, the method comprising
- providing (310) a fibrous mat (100) according to claim 1, which carries a metabolome sample,
- subjecting (320) the fibrous mat (100) to a device configured for desorbing the metabolome sample
- desorbing (330) the metabolome sample.

11. A method (300) according to claim 10 wherein providing (310) the fibrous mat (100) comprises taking the metabolome sample using the fibrous mat.

12. A method (300) according to any of the claims 10 to 11 wherein the desorbing is followed by or combined with ionizing (330) of the metabolome sample.

13. A method (300) according to any of the claims 10 to 12 wherein the provided fibrous mat (100) is a mat according to any of the claims 2 to 8.

14. A method (310) for providing a fibrous mat (100) wherein providing (310) the fibrous mat comprises:
- solving at least one polymer components in a solvent wherein hydrophilic and hydrophobic moieties are present in the at least one polymer component,
- electrospinning of the at least one dissolved polymer component thereby forming a fibrous mat (100) comprising (a) an open network (115) of at least one polymer-based nanofiber (110), wherein the at least one nanofiber (110) is spun with a diameter ranging between 50 nm and 1500 nm, providing (b) a cover layer (125) at a top and/or at a bottom of the open network (115) of at least one nanofiber (110) to exclude particles with a mass larger than 3,32×10-24Kg (2000 Da) from interacting with the open network (115) of at least one nanofiber (110), by blocking and/or excluding access to these particles.

15. A method (310) according to claim 14 wherein the at least one polymer is spun such that interconnected pores are formed between the nanofibers (110) of the open network (115).

## Patentansprüche

1. Eine Fasermatte (100), die zur Verwendung bei der Entnahme einer Metabolomprobe und zur Verwendung bei der Desorption der Metabolomprobe konfiguriert ist, wobei die Fasermatte (100) ein offenes Netzwerk (115) aus mindestens einer polymerbasierten Nanofaser (110) umfasst, wobei der Durchmesser der mindestens einen Nanofaser (110) im Bereich zwischen 50 nm und 1500 nm liegt,
**dadurch gekennzeichnet, dass** eine Deckschicht (125) an einer Oberseite und/oder an einer Unterseite des offenen Netzwerks aus mindestens einer Nanofaser bereitgestellt ist, um Partikel mit einer Masse von größer als 3,32×10⁻²⁴ kg (2000 Da) von der Wechselwirkung mit dem offenen Netzwerk (115) aus mindestens einer Nanofaser (110) auszuschließen, indem der Zugriff auf diese Partikel blockiert und/oder ausgeschlossen wird.

2. Eine Fasermatte (100) nach Anspruch 1, wobei ein Verhältnis hydrophiler/hydrophober Anteile der mindestens einen Nanofaser (110) derart ausgewählt ist, dass eine Größenverformung der Fasermatte nach Exposition gegenüber Wasser weniger als 5 % beträgt.

3. Eine Fasermatte (100) nach Anspruch 2, wobei das Verhältnis hydrophiler/hydrophober Anteile im Bereich von 75/25 bis 10/90 liegt.

4. Eine Fasermatte (100) nach einem der vorstehenden Ansprüche, die zur Verwendung bei der Desorption und Ionisierung der Metabolomprobe konfiguriert ist.

5. Eine Fasermatte (100) nach einem der vorstehenden Ansprüche, die zur Verwendung bei der lasergestützten Desorption der Metabolomprobe konfiguriert ist.

6. Eine Fasermatte (100) nach einem der vorstehenden Ansprüche, die zur Verwendung bei der Diagnose der Metabolomprobe konfiguriert ist.

7. Eine Fasermatte (100) nach einem der vorstehenden Ansprüche, wobei die Fasermatte Nano- oder Mikropartikel (135) umfasst, die in das offene Netzwerk (115) der mindestens einen polymerbasierten Nanofaser, oder in die mindestens eine polymerbasierte Nanofaser eingebunden sind.

8. Eine Fasermatte (100) nach einem der vorstehenden Ansprüche, wobei die mindestens eine Nanofaser (110) ein oder mehrere Materialien umfasst, ausgewählt aus:
- einer Liste hydrophiler Polymere, die Polyacrylat, Polyacrylnitril, Polyvinylpyrrolidon umfasst, und/oder aus einer Liste hydrophober Polymere, die Polystyrol, Divinylbenzol, Polydimethylsiloxan, Polydivinylbenzol umfasst, und/oder aus einer Liste kombinierter hydrophober/hydrophiler Polymere, die Polydivinylbenzol-co-Polyvinylpyrrolidon, hydrophiles Divinylbenzol, vernetztes Polyvinylpyrrolidon umfasst.

9. Eine Probenahmevorrichtung (200), die eine Fasermatte (100) nach einem der vorstehenden Ansprüche und einen Träger (210) umfasst, an dem die Fasermatte gesichert ist.

10. Ein Verfahren (300) zur Desorption einer Metabolomprobe, wobei das Verfahren umfasst
- Bereitstellen (310) einer Fasermatte (100) nach Anspruch 1, die eine Metabolomprobe trägt,
- Unterziehen (320) der Fasermatte (100) einer Vorrichtung, die zum Desorbieren der Metabolomprobe konfiguriert ist,
- Desorbieren (330) der Metabolomprobe.

11. Ein Verfahren (300) nach Anspruch 10, wobei das Bereitstellen (310) der Fasermatte (100) das Entnehmen der Metabolomprobe unter Verwendung der Fasermatte umfasst.

12. Ein Verfahren (300) nach einem der Ansprüche 10 bis 11, wobei im Anschluss oder kombiniert mit dem Desorbieren ein Ionisieren (330) der Metabolomprobe erfolgt.

13. Ein Verfahren (300) nach einem der Ansprüche 10 bis 12, wobei die bereitgestellte Fasermatte (100) eine Matte nach einem der Ansprüche 2 bis 8 ist.

14. Ein Verfahren (310) zum Bereitstellen einer Fasermatte (100), wobei das Bereitstellen (310) der Fasermatte umfasst:
- Lösen von mindestens einer Polymerkomponente in einem Lösungsmittel, wobei in der mindestens einen Polymerkomponente hydrophile und hydrophobe Anteile vorhanden sind,
- Elektrospinnen der mindestens einen gelösten Polymerkomponente, wodurch eine Fasermatte (100) gebildet wird, die (a) ein offenes Netzwerk (115) aus mindestens einer polymerbasierten Nanofaser (110) umfasst, wobei die mindestens eine Nanofaser (110) mit einem Durchmesser im Bereich zwischen 50 nm und 1500 nm gesponnen wird, Bereitstellen (b) einer Deckschicht (125) an einer Oberseite und/oder an einer Unterseite des offenen Netzwerks (115) aus mindestens einer Nanofaser (110), um Partikel mit einer Masse von größer als 3,32x10-24 kg (2000 Da) von der Wechselwirkung mit dem offenen Netzwerk (115) aus mindestens einer Nanofaser (110) auszuschließen, indem der Zugriff auf diese Partikel blockiert und/oder ausgeschlossen wird.

15. Ein Verfahren (310) nach Anspruch 14, wobei das mindestens eine Polymer derart gesponnen wird, dass zwischen den Nanofasern (110) des offenen Netzwerks (115) miteinander verbundene Poren gebildet werden.

## Revendications

1. Un tapis fibreux (100) configuré pour être utilisé dans la prise d'un échantillon de métabolome et pour être utilisé dans la désorption de l'échantillon de métabolome, le tapis fibreux (100) comprenant un réseau ouvert (115) d'au moins une nanofibre à base de polymère (110), dans lequel le diamètre de ladite au moins une nanofibre (110) varie entre 50 nm et 1500 nm, **caractérisé en ce qu'**une couche de couverture (125) est prévue en haut et/ou en bas du réseau ouvert d'au moins une nanofibre pour exclure les particules ayant une masse supérieure à 3,32×10⁻²⁴Kg (2000 Da) de l'interaction avec le réseau ouvert (115) d'au moins une nanofibre (110) en bloquant et/ou en excluant l'accès à ces particules.

2. Un tapis fibreux (100) selon la revendication 1 dans lequel un rapport de fraction hydrophile/hydrophobe de ladite au moins une nanofibre (110) est sélectionné de telle sorte que la déformation du tapis fibreux est inférieure à 5 % en taille après exposition à l'eau.

3. Un tapis fibreux (100) selon la revendication 2, dans lequel le rapport de fraction hydrophile/hydrophobe varie de 75/25 à 10/90.

4. Un tapis fibreux (100) selon l'une quelconque des revendications précédentes configuré pour être utilisé dans la désorption et l'ionisation de l'échantillon de métabolome.

5. Un tapis fibreux (100) selon l'une quelconque des revendications précédentes configuré pour être utilisé dans la désorption assistée par laser de l'échantillon de métabolome.

6. Un tapis fibreux (100) selon l'une quelconque des revendications précédentes configuré pour être utilisé dans le diagnostic de l'échantillon de métabolome.

7. Un tapis fibreux (100) selon l'une quelconque des revendications précédentes, le tapis fibreux comprenant des nano- ou micro-particules (135) qui sont incorporées dans le réseau ouvert (115) d'au moins une nanofibre à base de polymère ou dans ladite au moins une nanofibre à base de polymère.

8. Un tapis fibreux (100) selon l'une quelconque des revendications précédentes dans lequel ladite au moins une nanofibre (110) comprend un ou plusieurs matériaux sélectionnés parmi : une liste de polymères hydrophiles comprenant le polyacrylate, le polyacrylonitrile, le polyvinylpyrrolidone, et/ou parmi une liste de polymères hydrophobes comprenant le polystyrène, le divinylbenzène, le polydiméthylsiloxane, le polydivinylbenzène, et/ou parmi une liste de polymères combinés hydrophobes/hydrophiles comprenant le polydivinylbenzène-co-polyvinylpyrrolidone, le divinylbenzène hydrophile, le polyvinylpyrrolidone réticulé.

9. Un dispositif d'échantillonnage (200) comprenant un tapis fibreux (100) selon l'une quelconque des revendications précédentes et un support (210) auquel le tapis fibreux est fixé.

10. Un procédé (300) pour la désorption d'un échantillon de métabolome, le procédé comprenant
- fournir (310) un tapis fibreux (100) selon la revendication 1, qui porte un échantillon de métabolome,
- soumettre (320) le tapis fibreux (100) à un dispositif configuré pour désorber l'échantillon de métabolome
- désorber (330) l'échantillon de métabolome.

11. Un procédé (300) selon la revendication 10 dans lequel fournir (310) le tapis fibreux (100) comprend prendre l'échantillon de métabolome en utilisant le tapis fibreux.

12. Un procédé (300) selon l'une quelconque des revendications 10 à 11 dans lequel la désorption est suivie par ou combinée avec l'ionisation (330) de l'échantillon de métabolome.

13. Un procédé (300) selon l'une quelconque des revendications 10 à 12 dans lequel le tapis fibreux fourni (100) est un tapis selon l'une quelconque des revendications 2 à 8.

14. Un procédé (310) pour fournir un tapis fibreux (100) dans lequel fournir (310) le tapis fibreux comprend :
- dissoudre au moins un composant polymère dans un solvant dans lequel des fractions hydrophiles et hydrophobes sont présentes dans ledit au moins un composant polymère, - électrofiler ledit au moins un composant polymère dissous formant ainsi un tapis fibreux (100) comprenant (a) un réseau ouvert (115) d'au moins une nanofibre à base de polymère (110), dans lequel ladite au moins une nanofibre (110) est filée avec un diamètre variant entre 50 nm et 1500 nm, fournissant (b) une couche de couverture (125) en haut et/ou en bas du réseau ouvert (115) d'au moins une nanofibre (110) pour exclure les particules ayant une masse supérieure à 3,32×10⁻²⁴Kg (2000 Da) de l'interaction avec le réseau ouvert (115) d'au moins une nanofibre (110), en bloquant et/ou en excluant l'accès à ces particules.

15. Un procédé (310) selon la revendication 14 dans lequel ledit au moins un polymère est filé de telle sorte que des pores interconnectés sont formés entre les nanofibres (110) du réseau ouvert (115).
